# FASCICULE DE BREVET EUROPEEN

(11) **EP 0 524 865 B1**
(45) Date de publication et mention de la délivrance du brevet: **08.04.1998**
(21) Numéro de dépôt: 92402081.1
(22) Date de dépôt: 17.07.1992
(51) Int. Cl.: G02F 1/35, C07C 215/16, C07C 211/52, C07C 219/06, C07C 239/00

(54) **Matériau organique à chaîne polymère notamment pour l'optique non linéaire monomères organiques intermédiaires dans sa préparation et leurs procédés de préparation**
Organisches Material mit Polymerkette, insbesondere für nichtlineare Optik, organische Monomeren-Zwischenprodukte zu seiner Herstellung und Verfahren zu seiner Herstellung
Organic material with polymeric chain especially for nonlinear optic, organic monomers intermediates in its preparation, and process for their preparation

(30) Priorité: 19.07.1991 FR 9109180
(43) Date de publication de la demande: 27.01.1993
(73) Titulaire: ALCATEL ALSTHOM COMPAGNIE GENERALE D'ELECTRICITE, 75008 Paris (FR)
(72) Inventeur: Barraud, Jean-Yves, F-75010 Paris (FR); Gervat, Sophie, F-78340 Les Clayes sous Bois (FR); Fauvarque, Jean-François, F-75006 Paris (FR); Ratovelomanana, Victorin, F-91170 Viry Chatillon (FR)
(74) Mandataire: Laroche, Danièle

(56) Documents cités:
- EP-A- 0 326 163
- WO-A-91/02018
- WO-A-91/02019
- US-A- 4 826 950
- CHEMICAL PHYSICS vol. 150, no. 1 , 15 Janvier 1991 , NORTH-HOLLAND pages 117 - 123 I.LEDOUX,J.ZYSS 'nonlinear optical properties of asymmetric polyphenyls...'
- CHEMICAL PHYSICS vol. 150, no. 1 , 15 Janvier 1991 , NORTH-HOLLAND pages 125 - 135 J.ZYSS,I.LEDOUX 'dimethylaminocyanobiphenyl.....'
- JOURNAL OF ORGANOMETALLIC CHEMISTRY vol. 390, no. 3 , 1990 , LAUSANNE(CH) pages 389 - 398 C.AMATORE 'Efficient palladium catalyzed synthesis of unsymmetrical donor-acceptor biaryls and polyaryls'
- CHEMICAL PHYSICS vol. 153, no. 3 , 1 Juin 1991 , NORTH -HOLLAND pages 457 - 464 M.BARZOUKAS 'Conformational dependence of the quadratic hyperpolarisabilities of a seres of push-pull diaryl acetylenes.....'
- JOURNAL OF THE AMERICAN CHEMICAL SOCIETY vol. 113, no. 20 , 1991 , USA pages 7658 - 7666 A.E.STIEGMAN 'The electronic structure and second order nonlinear optical properties of donor- acceptor acetylenes...'

## Description

La présente invention concerne un matériau organique utilisable notamment pour l'optique non linéaire, dont la molécule comprend dans sa structure un groupe donneur d'électrons et un groupe attracteur d'électrons formé par un radical nitro, cyano, sulfonyl ou nitroso, reliés par l'intermédiaire d'un radical transmetteur d'électrons possédant au moins un noyau aryle. Elle s'étend en outre à un procédé de préparation de ce matériau, à des monomères organiques constituant des produits intermédiaires dans sa préparation et à leurs procédés de préparation.

Les matériaux présentant des propriétés non linéaires en optique pouvant être utilisés pour de nombreuses applications électro-optiques : doubleurs de fréquence, bistables optiques, commutateurs, modulateurs, coupleurs directifs, amplificateurs paramétriques, etc...

On connaît un petit nombre de matériaux minéraux possédant des propriétés non linéaires en optique, mais leur mise en oeuvre est difficile. C'est pourquoi on s'est intéressé à des matériaux organiques à propriétés non linéaires.

La demanderesse a déjà proposé comme matériaux organiques pour l'optique non linéaire dans les documents WO-A/01723, WO-A/01724 et EP-A-0424829 des dérivés cyanés du biphényle, des dérivés nitrés ou cyanés du thiophène ou du furanne ou des dérivés cyanés du triphényle ou du tétraphényle. Leur élaboration fait appel à la cristallogénèse en solution et exige à chaque étape des contrôles physco-chimiques difficiles.

Il est décrit dans le document US-A-4826950 un matériau organique pour l'optique non linéaire, comprenant un donneur d'électrons comportant un azote trivalent couplé avec un polyester aromatique, notamment téréphtalique, par l'intermédiaire de deux radicaux OH, à radical transmetteur d'électrons formé par un groupe biphényle, substitué par un groupe nitro ou cyano jouant le rôle d'attracteur d'électrons. Il est cependant souhaitable d'obtenir ces matériaux présentant des températures de transition vitreuse plus élevées, et des propriétés encore améliorées en optique non linéaire.

La présente invention a pour but de procurer de nouveaux matériaux organiques de fabrication facile, avec un bon rendement, possédant des températures de transition vitreuses assez élevées, supérieures à 100°C et même 120°C, et de bonnes propriétés électro-optiques, présentant peu de risques de claquage en utilisation, grâce à l'absence de traces de métaux. De tels matériaux sont isotropes et peuvent être déposés en film mince sur d'autres substrats, tels que As-Ga ou le silicium.

Le matériau organique selon l'invention est caractérisé en ce que son groupe donneur d'électrons comprend un monomère organique contenant un atome d'oxygène ou de soufre divalent, ou d'azote trivalent, couplé avec un polymère organique par condensation d'au moins deux radicaux - OH, - NH, ou -NH₂ avec un maillon de la chaîne du polymère, et en ce que son radical transmetteur d'électrons est un groupe tolane, le polymère organique de son donneur d'électrons étant choisi dans le groupe formé par les polyuréthannes, les polyépoxydes, les polyéthers, les polyesters, les polycarbonates, les polyurées, les polyuréthannes-urées et leurs copolymères.

Le procédé de préparation d'un matériau organique selon l'invention, à polymère du groupe formé par les polyuréthannes, les polyurées et les polyépoxydes, est caractérisé en ce que l'on condense un dérivé aryle à substituants nitro, cyano, sulfonyl ou nitroso d'une part, N-alkanolamino, N,N' - dialkanolamino ou N,N' - di (alkylamine) amino d'autre part, avec un isocyanate aromatique ou cyclanique ou un- diépoxyde.

Selon un procédé préféré de fabrication du monomère organique, on fait réagir le chlorure d'iode sur une - 4 - N,N' (di (O-acyl) alkanol aniline, on fait réagir la 4 - iodo - 4' - N,N' - (di (O-acyl) alkanol) aniline formée sur le 4 - cyanophénylacétylène en présence de dichlorobis (triphénylphosphine) palladium, d'iodure cuivreux et de trialkylamine, et on désacyle le 4 - cyano - 4' - N,N' - (di (O-acyl) alkanol) amino obtenu par le méthanol et la méthylate de sodium pour obtenir un 4 - cyano - 4' -N,N' dialkanolaminotolane, ou l'on effectue les réactions correspondantes à partir de 4 - N ( (O-acyl) alkanol) aniline ou de 4 - N,N' - (di (N-acyl) alkylamine) aniline.

Les composés constitués par les 4 - cyano - 4' - N,N' - di (O-acyl) alkanol) aminotolanes, les 4 - cyano - 4' - N (O-acyl) alkanol) aminotolanes, les 4 - cyano - 4' - N,N' (di (N-acyl) alkylamine) aminotolanes, les 4 - cyano - 4' - N,N' - dialkanolaminotolanes, les 4 - cyano - 4' - N (alkanolaminotolanes et les 4 - cyano - 4' - N,N' - (di (N-acyl) alkylamine) aminotolanes, et notamment le 4 - cyano - 4' - N,N' - di (O -acétyl) éthanol) aminotolane, le 4 - cyano - 4' - N ((O-acétyl) éthanol) aminotolane, le 4 - cyano - 4' - N,N' - (di (N-acétyl) éthanolamine) aminotolane, le 4 - cyano - 4' - N,N' - (diéthanolaminotolane, le 4 - cyano - 4' - N (O- acétyl) éthanol) aminotolane et le 4 - cyano - 4' - N,N' (di (N-acétyl) éthylamine) aminotolane sont nouveaux, et font à ce titre partie de l'invention.

Il est décrit ci-après à titre d'exemple un procédé de préparation d'un monomère de l'invention.

### Exemple 1 - Synthèse du 4 - cyano - 4' - N,N' - (diéthanol) aminotolane

a) préparation du 4 - cyano - 4' - N,N' (di (O-acétyl) éthanol) aminotolane.
   7,8 g (20 m mol) de 4 - iodo - 4' - N,N' - (di (O-acétyl) éthanol) aniline sont mis en solution dans 40 ml de benzène dégazé. 280 mg (2 %) de dichloro bis triphénylphosphine) palladium sont ajoutés, ainsi que 3 g (24 m mol) de 4 - cyano phénylacétylène (décrit dans Synthesis, 1980, p. 627). 76 mg (2 %) de CuI et 8 ml de triéthylamine. Après 5 heures de réaction à température ambiante, la solution est lavée par une solution saturée de chlorure d'ammonium. L'évaporation du solvant après séchage fournit 6,2 g (79 %) de produit brut, purifié sur silice.
b) préparation du 4 - cyano - 4' - N,N' - diéthanolamino tolane :
   5 g (13 m mol) du produit obtenu ci-dessus sont mis en solution dans 10 ml de méthanol sec. 26 ml d'une solution méthanolique centimolaire de méthylate de sodium sont ajoutés. Quand la réaction est terminée, (1h), le solvant est évaporé. On obtient 3,6 g de produit (92 %).

L'ensemble de cette synthèse est schématisé par le tableau ci-dessous.

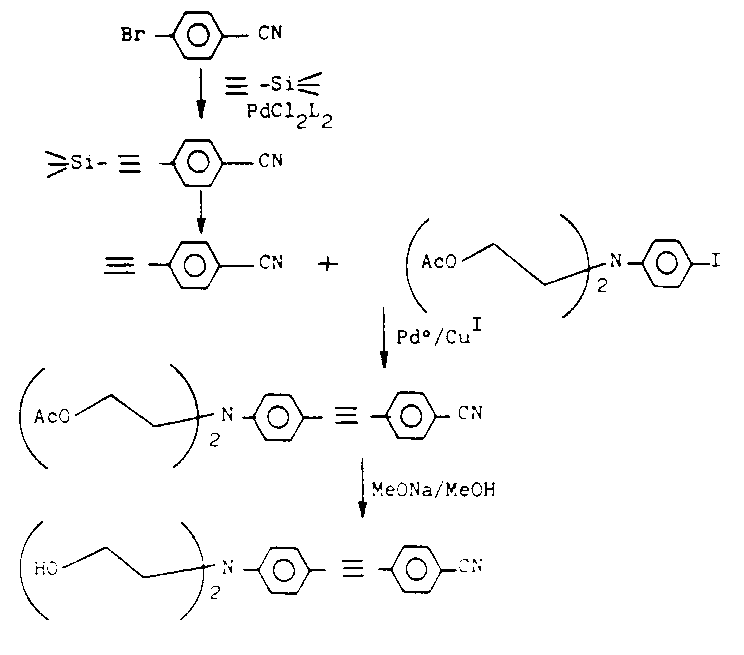

Grâce à leur structure les molécules actives précédentes de monomère sont facilement incorporées dans une matrice polymérique.

On fait réagir les diols des monomères sur des molécules difonctionnelles, telles que les diisocyanates aromatiques cu cyclaniques pour obtenir des polyuréthannes, ou des diépoxydes pour obtenir des polyépoxydes.

On fait par exemple réagir le 4 - cyano - 4' - N,N' - diéthanol aminotolane sur le toluène diisocyanate - 2,4 selon le schéma réactionnel ci-dessous, dans lequel - (T) - représente un radical transmetteur TDI le toluène diisocyanate

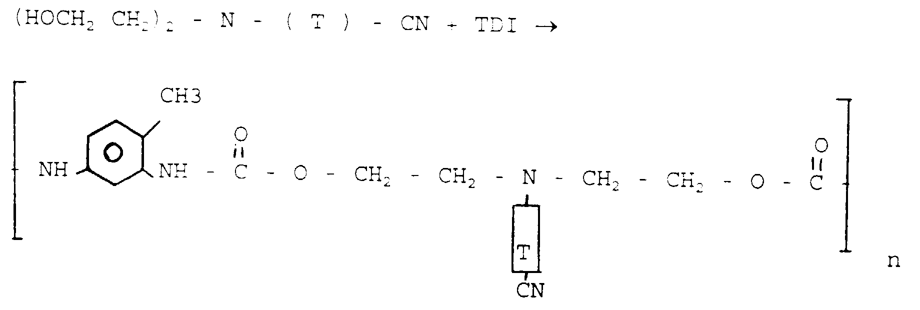

Ces matériaux sont ensuite déposés en couche mince entre deux électrodes. Ils sont mis en solution dans un solvant approprié et déposés à la tournette sous forme de films de quelques micromètres d'épaisseur, de manière connue. Le solvant est évaporé avant le dépôt de la seconde électrode.

Les matériaux polymères sans molécule active sont susceptibles de servir de tampons d'indice de réfraction au système de guidage d'ondes.

Les propriétés électro-optiques des matériaux isotropes selon l'invention se manifestent après application d'un champ électrique à une température voisine de leur température de transition vitreuse (supérieure à 120°C). On laisse refroidir le matériau en maintenant la tension de polarisation. Les propriétés acquises par le matériau se conservent par la suite.

La caractérisation électro-optique des matériaux s'effectue sur un résonateur Fabry-Pérot.

Ainsi la mesure du coefficient r₁₃ a été effectuée sur le copolymère toluène diisocyanate - 4 - (N,N'- diéthanol) amino - β - nitrostyrène donne une valeur de 5 picomètres/V à 0,638 µm. Ce matériau présente une température de transition vitreuse Tg = 130°C.

## Revendications

1. Matériau organique, notamment pour l'optique non linéaire, dont la molécule comprend dans sa structure un groupe donneur d'électrons et au moins un groupe attracteur d'électrons formé par un radical nitro, cyano, sulfonyl ou nitroso, relié par l'intermédiaire d'un radical transmetteur d'électrons possédant au moins un noyau aryle, caractérisé en ce que le groupe donneur d'électrons comprend un monomère organique contenant un atome d'oxygène ou de soufre divalent, ou d'azote trivalent, couplé avec un polymère organique par condensation d'au moins deux radicaux -OH, -NH ou -NH2 avec un maillon de la chaîne du polymère, et en ce que son radical transmetteur d'électrons est un groupe tolane, le polymère organique de son donneur d'électrons étant choisi dans le groupe formé par les polyuréthannes, les polyépoxydes, les polyéthers, les polyesters, les polycarbonates, les polyurées, les polyuréthanne-urées et leurs copolymères.

2. Procédé de préparation d'un matériau organique selon la revendication 1, à polymère du groupe formé par les polyurées, les polyuréthannes et les polyépoxydes, caractérisé en ce que l'on condense un dérivé du tolane à substituants nitro, cyano, sulfonyl ou nitroso d'une part, N-alkanolamino, N,N'-dialkanolamino ou N,N'-di(alkylamine), amino d'autre part, avec un diisocyanate aromatique ou cyclanique ou un diépoxyde.

3. Procédé de fabrication d'un monomère organique selon la revendication 1, caractérisé en ce que l'on fait réagir le chlorure d'iode sur une 4 - N,N' - (di- O - acyl) alkanol) aniline, on fait réagir la 4 - icdo - 4' - N,N' - (di (O-acyl) alkanol) aniline formée sur le 4 - cyanophénylacétyléne en présence de dichlorobis (triphénylphosphine) palladium, d'iodure cuivreux et de trialkylamine, et on désacyle le 4 - cyano - 4' - N,N' - (di (O-acyl) alkanol) amino obtenu par le méthanol et la méthylate de sodium pour obtenir un 4 - cyano - 4' - N,N' dialkanolaminotolane, ou l'on effectue les réactions correspondantes à partir de 4 - N (O -acyl) alkanol) aniline ou de 4 - N,N' - (di (N-acyl) alkylamine) aniline.

4. Les nouveaux composés constitués par les 4 -cyano - 4' - N,N' - (di (O-acyl) alkanol) aminotolanes, les 4 - cyano - 4' - N (O-acyl) alkanol) aminotolanes, les 4 - cyano - 4' - N,N' (di (N -acyl) alkylamine) aminotolanes, les 4 - cyano - 4' - N,N' - dialkanolaminotolanes, les 4 - cyano - 4' - N (alkanolaminotolanes et les 4 - cyano - 4' - N,N' - (di (N-acyl) alkylamine) aminotolanes, et notamment le 4 - cyano - 4' - N,N' - di (O-acétyl) éthanol aminotolane, le 4 - cyano - 4' - N ((O-acétyl) éthanol) aminotolane, le 4 - cyano - 4' - N,N' - di (N-acétyl) éthanolamine) aminotolane, le 4 - cyano - 4' - N,N'- diéthanolaminotolane, le 4 - cyano - 4' - N (O-acétyl) éthanol) aminotclane et le 4 - cyano - 4' - N,N' (di (N-acétyl) éthylamine) aminotolane.

## Patentansprüche

1. Organisches Material, insbesondere für die nichtlineare Optik, dessen Molekül in seiner Struktur einen Elektronenspender und einen, durch einen Nitro-, Cyan- , Sulfonyl- oder Nitroso-Rest gebildeten Elektronenempfänger enthält, welche durch einen elektronenüberführenden Rest verbunden sind, der wenigstens einen Arylkern aufweist, **dadurch gekennzeichnet,** dass sein Elektronenspender ein organisches Monomer mit einem zweiwertigen Sauerstoff oder Schwefelatom oder einem dreiwertigen Stickstoffatom enthält, das mit einem organischen Polymer durch Kondensation wenigstens zweier OH-, NH- oder NH₂-Reste mit einem Glied der Polymerkette gekoppelt ist, und dadurch dass sein elektronenüberführender Rest eine Tolangruppe ist, wobei das organische Polymer seines Elektronenspenders aus der Gruppe, die durch die Polyurethane, die Polyepoxide, die Polyäthere, die Polyester, die Polykarbonate, die Polyharnstoffe, die Polyurethan-Harnstoffe und ihre Copolymere gebildet ist, ausgewählt wird.

2. Verfahren zum Herstellen eines organischen Materials gemäß Anspruch 1, mit einem Polymer aus der Gruppe der Polyurethane, der Polyharnstoffe und der Polyepoxide, dadurch gekennzeichnet, dass man ein Arylderivat mit Nitro-, Cyan-, Sulfonyl- oder Nitroso-Substitutionsgruppen einerseits, N-Alkanolamino, N,N'-Dialkanolamino oder N,N'-Dialkylaminamino-Substitutionsgruppen andererseits mit einem aromatischen oder cyclanischen Isocyanat oder einem Diepoxid kondensiert.

3. Verfahren zum Herstellen eines organischen Monomers gemäß Anspruch 1, dadurch gekennzeichnet, dass man Jodchlorid auf ein -4-N,N' (di (O-Acyl) Alkanol Anilin reagieren lässt, man das so gebildete 4-Jod-4'-N,N'-(Di (O-Acyl) Alkanol) Anilin auf 4-Cyanphenylacetylen in Anwesenheit von Dichlorbis (Triphenylphosphin) Palladium, von Kupfer (I) Jodid und von Trialkylamin reagieren lässt, man das erhaltene 4-Cyan-4'-N,N'-Di (O-Acyl) Alkanol) Amin mittels Methanol und Natriummethylat desacyliert, um ein 4-Cyan-4'-N,N' Dialkanolaminotolan zu erhalten, oder man die entsprechenden Reaktionen, von einem 4-N ( (O-Acyl) Alkanol Anilin oder einem 4-N,N'-(di (N-Acyl) Alkylamin) Anilin ausgehend, durchführt.

4. Die neuen Verbindungen, beinhaltend: 4-Cyan-4'-N, N'-di (O-Acyl) Alkanol) Aminotolane, die 4-Cyan-4'N (O-Acyl) Alkanol) Aminctolane, die 4-Cyan-4'-N,N' (di (N-Acyl) Alkylamin) Aminotolane, die 4-Cyan-4'-N, N'-Dialkanolaminotolane, die 4-Cyan-4'-N-Alkanolaminotolane und die 4-Cyan-4'-N,N'-di (N-Acyl Alkylamin) Aminotolane und insbesondere das 4-Cyan-4'-N,N'-di (O-Acetyl) Ethanol) Aminotolan, das 4-Cyan-4'-N ((O-Acetyl) Ethanol) Aminotolan, das 4-Cyan-4'-N,N'- (Di (N-Acetyl) Ethanolamin Aminotolan, das 4-Cyan-4' -N,N'-(Diethanolaminotolan, das 4-Cyan-4'-N (O-Acetyl) Ethanol) Aminotolan und das 4-Cyan-4'-N,N' (Di (N-Acetyl) Ethylamin) Aminotolan.

## Claims

1. An organic material, particularly for nonlinear optics, the molecule of which comprises in its structure an electron-donor group and at least one electron-attractor group formed by a nitro, cyano, sulfonyl or nitroso radical, connected via an electron-transmitter radical containing at least one aryl ring, characterized in that the electron-donor group comprises an organic monomer containing a divalent oxygen or sulfur atom, or a trivalent nitrogen atom, coupled with an organic polymer by condensation of at least two -OH, -NH or -NH₂ radicals with a link in the polymer chain, and in that its electron-transmitter radical is a tolane group, the organic polymer and its electron-donor being selected from the group formed by polyurethanes, polyepoxides, polyethers, polyesters, polycarbonates, polyureas, polyurethane-ureas and copolymers thereof.

2. A method of preparing the organic material of claim 1, containing a polymer from the group formed by polyureas, polyurethanes and polyepoxides, characterized in that a tolane derivative containing nitro, cyano, sulfonyl or nitroso substituents and N-alkanolamino, N,N'-dialkanolamino or N,N'-di(alkylamine)amino substituents is condensed with an aromatic or alicyclic diisocyanate or a diepoxide.

3. A method of producing an organic monomer according to claim 1, characterized in that iodine chloride is reacted with a 4-N,N'(di(O-acyl)alkanol)aniline, the 4-iodo-4'-N,N'-(di(O-acyl)alkanol)aniline formed is reacted with 4-cyanophenylacetylene in the presence of dichlorobis(triphenylphosphine) palladium, copper (II) iodide and a trialkylamine, and the 4-cyano-4'-N,N'-(di(O-acyl)alkanol) amino compound obtained is deacylated with methanol and sodium methylate to obtain a 4-cyano-4'-N,N'-dialkanolaminotolane, or corresponding reactions are carried out using 4-N((O-acyl)alkanol)aniline or 4-N,N'-(di(N-acyl)alkylamine)aniline.

4. Novel compounds constituted by
4-cyano-4'-N,N'-(di(O-acyl)alkanol)aminotolanes,
4-cyano-4'-N((O-acyl)alkanol)aminotolanes,
4-cyano-4'-N,N'(di(N-acyl)alkylamine)aminotolanes,
4-cyano-4'-N,N'-dialkanolaminotolanes,
4-cyano-4'-N(alkanolaminotolanes) and
4-cyano-4'-N,N'-(di(N-acyl)alkylamine)aminotolanes, and in particular
4-cyano-4'-N,N'-(di(O-acetyl)ethanol)aminotolane,
4-cyano-4'-N((O-acetyl)ethanol)aminotolane,
4-cyano-4'-N,N'-(di(N-acetyl)ethanolamine)aminotolane,
4-cyano-4'-N,N'-diethanolaminotolane,
4-cyano-4'-N-((O-acetyl)ethanol)aminotolane and
4-cyano-4'-N,N'(di(N-acetyl)ethylamine)aminotolane
